# EUROPÄISCHE PATENTSCHRIFT

(11) **EP 0 708 088 B1**
(45) Veröffentlichungstag und Bekanntmachung des Hinweises auf die Patenterteilung: **24.03.1999**
(21) Anmeldenummer: 95116265.0
(22) Anmeldetag: 16.10.1995
(51) Int. Cl.: C07C 317/46, A61K 31/155

(54) **Alkyl-5-methylsulfonyl-benzoyl-guanidin-Derivate**
Alkyl-5-methylsulphonyl-benzoyl-guanidine derivatives
Dérivés d'alkyle-5-méthylesulphonyle-benzoyle-guanidine

(30) Priorität: 22.10.1994 DE 4437874
(43) Veröffentlichungstag der Anmeldung: 24.04.1996
(73) Patentinhaber: MERCK PATENT GmbH, 64293 Darmstadt (DE)
(72) Erfinder: Gericke, Rolf, Dr., D-64342 Seeheim (DE); Dorsch, Dieter, Dr., D-64372 Ober-Ramstadt (DE); Baumgarth, Manfred, Dr., D-64297 Darmstadt (DE); Minck, Klaus-Otto, Dr., D-64372 Ober-Ramstadt (DE); Beier, Norbert, Dr., D-64354 Reinheim (DE)

(56) Entgegenhaltungen:
- EP-A- 0 589 336
- EP-A- 0 602 523

## Beschreibung

Die Erfindung betrifft Benzoylguanidin-Derivate der Formel I 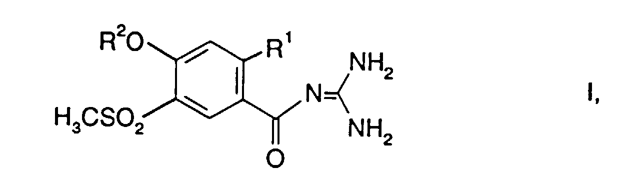 worin
- R¹: Methyl oder Ethyl und
- R²: H, einen geradkettigen oder verzweigten C₁-C₆-Alkyl- oder Alkenyl-Rest, C₃-C₇-Cycloalkyl, Benzyl oder unsubstituiertes oder ein-, zwei- oder dreifach durch Methyl, Methoxy, Amino, F, Cl, Br oder CF₃ substituiertes Phenyl
bedeuten,
sowie deren physiologisch unbedenkliche Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1992)) und die somit gute Antiarrhythmika darstellen. Sie eignen sich insbesondere zur Behandlung von Arrhythmien, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Darüber hinaus kennt man strukturell ähnliche Verbindungen beispielsweise aus EP 04 16 499.

Andere Benzoylguanidine mit antiarrhythmischer Wirkung, die jedoch in der ortho-Stellung zur Benzoyl-guanidino-Gruppe keine Substitution aufweisen, sind in der EP-A-602523 und in der EP-A-589336 beschrieben.

Gegenstand der Erfindung sind Verbindungen der Formel I sowie ihre physiologisch unbedenklichen Salze.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zell-proliferation bedingten Erkrankungen wie Arteriosklerose, diabetischen Spätkomplikationen, Tumorerkrankungen, fibrotischen Erkrankungen, insbesondere von Lunge, Leber und Nieren sowie Organhypertrophien und -hyperplasien eingesetzt werden. Darüber hinaus eignen sich die Substanzen zur diagnostischen Anwendung zur Erkennung von Krankheiten, die von einer gesteigerten Aktivität des Na+/H+-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von N. Escobales and J. Figueroa in J. Membrane Biol. 120, 41-49 (1991) oder von L. Counillon, W. Scholz, H.J. Lang und J. Pouysségur in Mol. Pharmacol. 44, 1041-1045 (1993) angegeben werden.

Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe Verwendung finden.

R² bedeutet vorzugsweise Methyl, Ethyl oder Phenyl, ferner aber auch bevorzugt Benzyl oder Cycloalkyl mit 3-, 5- oder 6 C-Atomen, insbesondere also Cyclopropyl, Cyclopentyl oder Cyclohexyl. Sofern R² Phenyl bedeutet, so ist dieses vorzugsweise unsubstituiert oder einfach durch Fluor oder Chlor substituiert.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Verbindungen der Formel I sind:
N-Diaminomethylen-2-methyl-4-phenoxy-5-methylsulfonylbenzamid;
N-Diaminomethylen-2-ethyl-4-phenoxy-5-methylsulfonylbenzamid;
N-Diaminomethylen-2-methyl-4-hydroxy-5-methylsulfonylbenzamid;
N-Diaminomethylen-2-ethyl-4-hydroxy-5-methylsulfonylbenzamid;
N-Diaminomethylen-2-methyl-4-benzyloxy-5-methylsulfonylbenzamid;
N-Diaminomethylen-2-ethyl-4-benzyloxy-5-methylsulfonylbenzamid;
N-Diaminomethylen-2-ethyl-4-benzyloxy-5-methylsulfonylbenzamid;
N-Diaminomethylen-2-ethyl-4-methoxy-5-methylsulfonylbenzamid;
N-Diaminomethylen-2-ethyl-4-cyclopropyloxy-5-methylsulfonylbenzamid;
N-Diaminomethylen-2-ethyl-4-cyclopentyloxy-5-methylsulfonylbenzamid;
N-Diaminomethylen-2-ethyl-4-cyclohexyloxy-5-methylsulfonylbenzamid.

Gegenstand der Erfindung ist ferner ein Verfahren zur Herstellung der Verbindungen der Formel I gemäß Anspruch 1, sowie von deren Salzen, dadurch gekennzeichnet, daß man eine Verbindung der Formel II 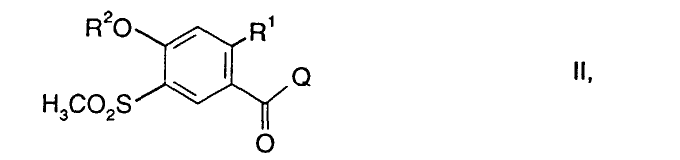 worin R¹ und R² die zuvor angegebenen Bedeutungen haben
und
- Q: Cl, Br, OA, O-CO-A, O-CO-Ph, OH oder eine andere reaktionsfähige veresterte OH-Gruppe bzw. leicht nucleophil substituierbare Abgangsgruppe und
- A: Alkyl mit 1-6 C-Atomen bedeutet,
mit Guanidin umsetzt, oder daß man ein Benzoylguanidin der Formel III 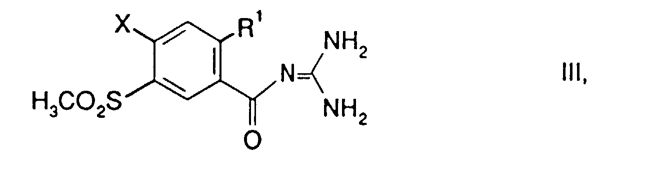 worin R¹ die zuvor angegebenen Bedeutungen besitzt,
und
- X: F, Cl, Br, I oder eine andere geeignete Abgangsgruppe bedeutet,
mit einem Alkohol bzw. Phenol der Formel IV

R²-OH IV

worin
- R²: die angegebene Bedeutung besitzt,
gegebenenfalls nach vorheriger Überführung in ein Alkoholat,
umsetzt,
oder daß man einen Alkohol (Phenol) der Formel I, worin R² = H und R¹ die angegebene Bedeutung besitzt, mit einer Verbindung der Formel R²-X, worin R² und X die angegebenen Bedeutungen besitzen, umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man eine erhaltene Base der Formel I durch Behandeln mit einer Säure in eines ihrer Salze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden Verbindungen der Formel I hergestellt, indem man ein aktiviertes Carbonsäurederivat der Formel II, wobei Q besonders bevorzugt Cl oder -O-CH₃ ist, mit Guanidin umsetzt. Besonders geeignet sind auch Reaktionsvarianten, bei denen die freie Carbonsäure II (Q = OH) in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol, Dicyclohexylcarbodiimid oder die Mukayama-Variante (Angew. Chem. 91, 788-812 (1979)).

Die Carbonsäuren und Carbonsäurederivate der Formel II sind in der Regel bekannt. Sie werden insbesondere hergestellt, indem man ein entsprechendes 2-Alkyl-4-halogen-5-methylsulfonylbenzoesäurederivat mit einem Alkoholat oder einem Phenolat, welches aus einem Alkohol oder Phenol der Formel IV ableitbar ist, umsetzt.

Die Umsetzung erfolgt in Analogie zu der Reaktion der Verbindungen III und IV. Sie wird nachstehend beschrieben.

Die Umsetzung eines reaktionsfähigen Carbonsäurederivates der Formel II mit Guanidin erfolgt in an sich bekannter Weise vorzugsweise in einem protischen oder aprotischen polaren oder unpolaren inerten organischen Lösungsmittel.

Geeignete Lösungsmittel werden nachfolgend für die Umsetzung der Verbindungen III und IV genannt. Besonders bevorzugte Solventien sind jedoch Methanol, THF, Dimethoxyethan, Dioxan oder daraus herstellbare Gemische sowie Wasser. Als Reaktionstemperatur sind beispielsweise Temperaturen zwischen 20° und dem Siedepunkt des Lösungsmittels geeignet. Die Reaktionszeiten liegen zwischen 5 Min. und 12 Std. Es ist zweckmäßig, bei der Reaktion einen Säurefänger einzusetzen. Hierzu eignen sich jegliche Arten von Basen, die die Reaktion selbst nicht stören. Besonders geeignet ist jedoch die Verwendung von anorganischen Basen wie Kaliumcarbonat oder von organischen Basen wie Triethylamin oder Pyridin oder aber ein Überschuß des Guanidins.

Verbindungen der Formel I gemäß Anspruch 1 können ferner hergestellt werden, indem man ein Benzoylguanidin der Formel III mit einer Verbindung der Formel IV umsetzt. Die Ausgangsstoffe der Formel III können durch Umsetzung von entsprechend substituierten Benzoesäuren oder daraus ableitbaren reaktionsfähigen Säurederivaten, wie z.B. Säurehalogeniden, Estern oder Anhydriden, mit Guanidin unter Reaktionsbedingungen wie sie für die Amidherstellung an sich bekannt und allgemein üblich sind, hergestellt werden. Besonders geeignet sind wiederum solche Reaktionsvarianten, wie sie zuvor für die Umsetzung von Verbindung II mit Guanidin angegeben wurden.

Die Herstellung der Verbindung II sowie die Umsetzung der Verbindung III mit einer Verbindung der Formel IV erfolgt in an sich bekannter Weise, bevorzugt in einem protischen oder aprotischen polaren inerten organischen Lösungsmittel.

Bei der Herstellung von II oder bei der Umsetzung von III mit IV ist es ebenfalls zweckmäßig, in Gegenwart einer Base oder mit einem Überschuß der basischen Komponente zu arbeiten. Als Basen eignen sich bevorzugt z.B. Alkalimetall- oder Erdalkalimetallhydroxide, -carbonate, -alkoholate oder organische Basen wie Triethylamin oder Pyridin, die auch im Uberschuß angewendet werden und dann gleichzeitig als Lösungsmittel dienen können.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Ebenso können die Verbindungen der Formel I erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer OH-Gruppe eine OR"-Gruppe tragen, worin R" eine Hydroxyschutzgruppe bedeutet.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCI in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfembare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Eine Base der Formel I kann ferner mit einer Säure in das zugehörige Säureadditionssalz übergeführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure. Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Himödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankunken, Fibrosen sowie Organhypertrophien und -hyperplasien, insbesondere bei Erkrankungen der Prostata.

Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheilszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

Man kocht eine Lösung von 1,4 g 2-Methyl-4-ethoxy-5-methylsulfonylbenzoesäuremethylester [erhältlich durch Umsetzung von 2-Methyl-4-chlor-5-methylsulfonyl-benzoesäure mit Natrium-ethanolat und anschließende Veresterung] und 1,5 g Guanidin in 50 ml Methanol fünf Stunden und entfemt anschließend das Lösungsmittel. Der Rückstand wird mit Wasser behandelt, das verbleibende Kristallisat abgesaugt und mit verdünnter Natronlauge behandelt. Man filtriert den festen Rückstand ab, kristallisiert aus Ethanol um und erhält N-Diaminomethylen-2-methyl-4-ethoxy-5-methylsulfonyl-benzamid, F. 201-203°.

Analog erhält man durch Umsetzung von Guanidin
mit 2-Ethyl-4-ethoxy-5-methylsulfonyl-benzoesäuremethylester das N-Diaminomethylen-2-ethyl-4-ethoxy-5-methylsulfonyl-benzamid;
mit 2-Methyl-4-isopropoxy-5-methylsulfonyl-benzoesäuremethylester das N-Diaminomethylen-2-methyl-4-isopropoxy-5-methylsulfonyl-benzamid, F. 185-188°;
mit 2-Methyl-4-tert.-butoxy-5-methylsulfonyl-benzoesäuremethylester das N-Diaminomethylen-2-methyl-4-tert.-butoxy-5-methylsulfonyl-benzamid, F. 205-207°;
mit 2-Methyl-4-butoxy-5-methylsulfonyl-benzoesäuremethylester das N-Diaminomethylen-2-methyl-4-butoxy-5-methylsulfonyl-benzamid;
mit 2-Methyl-4-(2-butoxy)-5-methylsulfonyl-benzoesäuremethylester das N-Diaminomethylen-2-methyl-4-(2-butoxy)-5-methylsulfonyl-benzamid;
mit 2-Methyl-4-cyclopentoxy-5-methylsulfonyl-benzoesäuremethylester das N-Diaminomethylen-2-methyl-4-cyclopentoxy-5-methylsulfonyl-benzamid, F. 238-241°;
mit 2-Methyl-4-(2-pentoxy)-5-methylsulfonyl-benzoesäuremethylester das N-Diaminomethylen-2-methyl-4-(2-pentoxy)-5-methylsulfonyl-benzamid;
mit 2-Methyl-4-(3-pentoxy)-5-methylsulfonyl-benzoesäuremethylester das N-Diaminomethylen-2-methyl-4-(3-pentoxy)-5-methylsulfonyl-benzamid;
mit 2-Methyl-4-cydohexyloxy-5-methylsulfonyl-benzoesäuremethylester das N-Diaminomethylen-2-methyl-4-cydohexyloxy-5-methylsulfonyl-benzamid, F. 224-226°;
mit 2-Methyl-4-cyclopropoxy-5-methylsulfonyl-benzoesäuremethylester das N-Diaminomethylen-2-methyl-4-cyclopropoxy-5-methylsulfonyl-benzamid;
mit 2-Ethyl-4-methoxy-5-methylsulfonyl-benzoesäuremethylester das N-Diaminomethylen-2-ethyl-4-methoxy-5-methylsulfonyl-benzamid, F. 192-195°;
mit 2-Ethyl-4-isopropoxy-5-methylsulfonyl-benzoesäuremethylester das N-Diaminomethylen-2-ethyl-4-isopropoxy-5-methylsulfonyl-benzamid;
mit 2-Ethyl-4-propoxy-5-methylsulfonyl-benzoesäuremethylesler das N-Diaminomethylen-2-ethyl-4-propoxy-5-methylsulfonyl-benzamid;
mit 2-Ethyl-4-butoxy-5-methylsulfonyl-benzoesäuremethylester das N-Diaminomethylen-2-ethyl-4-butoxy-5-methylsulfonyl-benzamid;
mit 2-Ethyl-4-(2-butoxy)-5-methylsulfonyl-benzoesäuremethylester das N-Diaminomethylen-2-ethyl-4-(2-butoxy)-5-methylsulfonyl-benzamid;
mit 2-Ethyl-4-tert.-butoxy-5-methylsulfonyl-benzoesäuremethylester das N-Diaminomethylen-2-methyl-4-tert.-butoxy-5-methylsulfonyl-benzamid;
mit 2-Ethyl-4-pentoxy-5-methylsulfonyl-benzoesäuremethylesler das N-Diaminomethylen-2-ethyl-4-pentoxy-5-methylsulfonyf-benzamid;
mit 2-Ethyl-4-(2-pentoxy)-5-methylsulfonyl-benzoesäuremethylester das N-Diaminomethylen-2-ethyl-4-(2-pentoxy)-5-methylsulfonyl-benzamid;
mit 2-Ethyl-4-(3-pentoxy)-5-methylsulfonyl-benzoesäuremethylester das N-Diaminomethylen-2-ethyl-4-(3-pentoxy)-5-methylsulfonylbenzamid;
mit 2-Ethyl-4-cyclopropoxy-5-methylsulfonyl-benzoesäuremethylester das N-Diaminomethylen-2-ethyl-4-cydopropoxy-5-methylsulfonyl-benzamid;
mit 2-Ethyl-4-cyclopentoxy-5-methylsulfonyl-benzoesäuremethylester das N-Diaminomethylen-2-ethyl-4-cyclopentoxy-5-methylsulfonyl-benzamid;
mit 2-Ethyl-4-cyclohexyloxy-5-methylsulfonyl-benzoesäuremethylester das N-Diaminomethylen-2-ethyl-4-cyclohexyloxy-5-methylsulfonyl-benzamid.

### Beispiel 2

3,0 g N-Diaminomethylen-2-ethyl-4-fluor-5-methylsulfonyl-benzamid [erhältlich durch Umsetzung von 2-Ethyl-4-fluor-5-methylsulfonyl-benzoesäuremethylester mit Guanidin] und 1,5 g Natrium-methylat werden in 25 ml Methanol 16 Stunden gekocht. Nach üblicher Aufarbeitung erhält man N-Diaminomethylen-2-ethyl-4-methoxy-5-methylsulfonyl-benzamid, F. 192-195°.

Analog erhält man durch Umsetzung von Natriummethylat
mit N-Diaminomethylen-2-methyl-4-chlor-5-methylsulfonyl-benzamid das N-Diaminomethylen-2-methyl-4-methoxy-5-methylsulfonyl-benzamid, F. 189-190°.

### Beispiel 3

700 mg N-Diaminomethylen-2-ethyl-4-methoxy-5-methylsulfonyl-benzamid [erhältlich nach Bsp. 2] werden in 50 ml Wasser suspendiert und unter Rühren mit 1,8 ml 1 n HCI versetzt. Nach Filtration und Lyophilisation erhält man N-Diaminomethylen-2-ethyl-4-methoxy-5-methylsulfonyl-benzamid-Hydrochlorid, F. 217-220°.

Analog erhält man aus der freien Base das
N-Diaminomethylen-2-methyl-4-ethoxy-5-methylsulfonyl-benzamid, Hydrochlorid, F. 218-219°;
N-Diaminomethylen-2-methyl-4-isopropoxy-5-methylsulfonyl-benzamid, Hydrochlorid, F. 216-220°;
N-Diaminomethylen-2-methyl-4-cyclopentoxy-5-methylsulfonyl-benzamid, Hydrochlorid, F. > 250°;
N-Diaminomethylen-2-methyl-4-cyclohexyloxy-5-methylsulfonyl-benzamid, Hydrochlorid, F. 185-187°;
N-Diaminomethylen-2-methyl-4-benzyloxy-5-methylsulfonyl-benzamid, Hydrochlorid, amorph;
N-Diaminomethylen-2-methyl-4-methoxy-5-methylsulfonyl-benzamid, Hydrochlorid, amorph.

### Beispiel 4

1,8 g 2-Methyl-4-phenoxy-5-methylsulfonyl-benzoesäure [erhältlich durch Umsetzung von 2-Methyl-4-chlor-5-methylsulfonyl-benzoesäure mit Natriumphenolat bei 180°] werden zusammen mit 25 ml SOCl₂ drei Stunden gekocht. Die klare Lösung wird eingeengt und der Rückstand in 20 ml Ethylenglykoldimethylether gelöst. Diese Reaktionsmischung wird langsam bei Raumtemperatur zu 2,5 g Guanidin, gelöst in 30 ml Ethylenglykoldimethylether, hinzugefügt und drei Stunden nachgerührt. Anschließend wird die Lösung zur Hälfte eingeengt, mit 50 ml Wasser versetzt, angesäuert und wie üblich aufgearbeitet. Man erhält N-Diaminomethylen-2-methyl-4-phenoxy-5-methylsulfonyl-benzamid, F. 248-250°.

Nach Suspendieren der Base in 50 ml Wasser und Behandlung mit 1,8 ml 1 n HCI unter Rühren erhält man im Anschluß an Filtration und Lyophilisation das N-Diaminomethylen-2-methyl-4-phenoxy-5-methylsulfonyl-benzamid; Hydrochlorid, F. > 250°.

### Beispiel 5

Analog Beispiel 4 erhält man durch Umsetzung von 2-Methyl-4-(2-chlor-phenoxy)-5-methylsulfonyl-benzoesäure [erhältlich durch Umsetzung von 2-Methyl-4-chlor-5-methylsulfonyl-benzoesäure mit Natrium-2-chlorphenolat bei 180°] mit SOCl₂ und anschließend mit Guanidin das N-Diamino-methylen-2-methyl-4-(2-chlor-phenoxy)-5-methylsulfonyl-benzamid, F. 188-191°.

Analog erhält man
mit 2-Methyl-4-(3-chlor-phenoxy)-5-methylsulfonyl-benzoesäure das N-Diaminomethylen-2-methyl-4-(3-chlor-phenoxy)-5-methylsulfonylbenzamid, F. 205-207°;
mit 2-Methyl-4-(4-chlor-phenoxy)-5-methylsulfonyl-benzoesäure das N-Diaminomethylen-2-methyl-4-(4-chlor-phenoxy)-5-methylsulfonyl-benzamid, F. 219-221°;
mit 2-Methyl-4-(2,4-dichlor-phenoxy)-5-methylsulfonyl-benzoesäure das N-Diaminomethylen-2-methyl-4-(2,4-dichlor-phenoxy)-5-methylsulfonyl-benzamid;
mit 2-Methyl-4-(2-fluor-phenoxy)-5-methylsulfonyl-benzoesäure das N-Diaminomethylen-2-methyl-4-(2-fluor-phenoxy)-5-methylsulfonyl-benzamid;
mit 2-Methyl-4-(3-fluor-phenoxy)-5-methylsulfonyl-benzoesäure das N-Diaminomethylen-2-methyl-4-(3-fluor-phenoxy)-5-methylsulfonyl-benzamid;
mit 2-Methyl-4-(4-fluor-phenoxy)-5-methylsulfonyl-benzoesäure das N-Diaminomethylen-2-methyl-4-(4-fluor-phenoxy)-5-methylsulfonyl-benzamid;
mit 2-Methyl-4-(2,4-difluor-phenoxy)-5-methylsulfonyl-benzoesäure das N-Diam inomethylen-2-methyl-4-(2,4-difluor-phenoxy)-5-methylsulfonyl-benzamid;
mit 2-Methyl-4-benzyloxy-5-methylsulfonyl-benzoesäure das N-Diaminomethylen-2-methyl-4-benzyloxy-5-methylsulfonyl-benzamid, F. 212-217°;
mit 2-Methyl-4-(3-methoxy-phenoxy)-5-methylsulfonyl-benzoesäure das N-Diaminomethylen-2-methyl-4-(3-methoxy-phenoxy)-5-methylsulfonyl-benzamid;
mit 2-Methyl-4-(4-methoxy-phenoxy)-5-methylsulfonyl-benzoesäure das N-Diaminomethylen-2-methyl-4-(4-methoxy-phenoxy)-5-methylsulfonyl-benzamid;
mit 2-Methyl-4-(2,4-dimethoxy-phenoxy)-5-methylsulfonyl-benzoesäure das N-Diaminomethylen-2-methyl-4-(2,4-dimethoxy-phenoxy)-5-methylsulfonyl-benzamid;
mit 2-Methyl-4-(2-methoxy-phenoxy)-5-methylsulfonyl-benzoesäure das N-Diaminomethylen-2-methyl-4-(2-methoxy-phenoxy)-5-methylsulfonyl-benzamid;
mit 2-Methyl-4-(3-trifluormethyl-phenoxy)-5-methylsulfonyl-benzoesäure das N-Diaminomethylen-2-methyl-4-(3-trifluormethyl-phenoxy)-5methylsulfonyl-benzamid;
mit 2-Methyl-4-(4-trifluormethyl-phenoxy)-5-methylsulfonyl-benzoesäure das N-Diam inomethylen-2-methyl-4-(4-trifluormethyl-phenoxy)-5-methylsulfonyl-benzamid;
mit 2-Methyl-4-(2-trifluormethyl-phenoxy)-5-methylsulfonyl-benzoesäure das N-Diaminomethylen-2-methyl-4-(2-trifluormethyl-phenoxy)-5-methylsulfonyl-benzamid;
mit 2-Methyl-4-(3-methyl-phenoxy)-5-methylsulfonyl-benzoesäure das N-Diaminomethylen-2-methyl-4-(3-methyl-phenoxy)-5-methylsulfonyl-benzamid;
mit 2-Methyl-4-(4-methyl-phenoxy)-5-methylsulfonyl-benzoesäure das N-Diaminomethylen-2-methyl-4-(4-methyl-phenoxy)-5-methylsulfonyl-benzamid;
mit 2-Methyl-4-(2,4-dimethyl-phenoxy)-5-methylsulfonyl-benzoesäure das N-Diaminomethylen-2-methyl-4-(2,4-dimethyl-phenoxy)-5-methylsulfonyl-benzamid;
mit 2-Methyl-4-(2-methyl-phenoxy)-5-methylsulfonyl-benzoesäure das N-Diam inomethylen-2-methyl-4-(2-methyl-phenoxy)-5-methylsulfonyl-benzamid.

### Beispiel 6

Analog Beispiel 4 erhält man durch Umsetzung von 2-Ethyl-4-(2-chlorphenoxy)-5-methylsulfonyl-benzoesäure [erhältlich durch Umsetzung von 2-Ethyl-4-chlor-5-methylsulfonyl-benzoesäure mit Natrium-2-chlorphenolat bei 180°] mit SOCl₂ und anschließend mit Guanidin das N-Diamino-methylen-2-ethyl-4-(2-chlor-phenoxy)-5-methylsulfonyl-benzamid.

Analog erhält man
mit 2-Ethyl-4-phenoxy-5-methylsulfonyl-benzoesäure das N-Diaminomethylen-2-ethyl-4-phenoxy-5-methylsulfonyl-benzamid;
mit 2-Ethyl-4-(3-chlor-phenoxy)-5-methylsulfonyl-benzoesäure das N-Diaminomethylen-2-ethyl-4-(3-chlor-phenoxy)-5-methylsulfonyl-benzamid;
mit 2-Ethyl-4-(4-chlor-phenoxy)-5-methylsulfonyl-benzoesäure das N-Diaminomethylen-2-ethyl-4-(4-chlor-phenoxy)-5-methylsulfonyl-benzamid;
mit 2-Ethyl-4-(2,4-dichlor-phenoxy)-5-methylsulfonyl-benzoesäure das N-Diaminomethylen-2-ethyl-4-(2,4-dichlor-phenoxy)-5-methylsulfonyl-benzamid;
mit 2-Ethyl-4-(2-fluor-phenoxy)-5-methylsulfonyl-benzoesäure das N-Diaminomethylen-2-ethyl-4-(2-fluor-phenoxy)-5-methylsulfonyl-benzamid;
mit 2-Ethyl-4-(3-fluor-phenoxy)-5-methylsulfonyl-benzoesäure das N-Diaminomethylen-2-ethyl-4-(3-fluor-phenoxy)-5-methylsulfonyl-benzamid;
mit 2-Ethyl-4-(4-fluor-phenoxy)-5-methylsulfonyl-benzoesäure das N-Diaminomethylen-2-ethyl-4-(4-fluor-phenoxy)-5-methylsulfonyl-benzamid;
mit 2-Ethyl-4-(2,4-difluor-phenoxy)-5-methylsulfonyl-benzoesäure das N-Diaminomethylen-2-ethyl-4-(2,4-difluor-phenoxy)-5-methylsulfonyl-benzamid;
mit 2-Ethyl-4-benzyloxy-5-methylsulfonyl-benzoesäure das N-Diaminomethylen-2-ethyl-4-benzyloxy-5-methylsulfonyl-benzamid;
mit 2-Ethyl-4-(3-methoxy-phenoxy)-5-methylsulfonyl-benzoesäure das N-Diaminomethylen-2-ethyl-4-(3-methoxy-phenoxy)-5-methylsulfonyl-benzamid;
mit 2-Ethyl-4-(4-methoxy-phenoxy)-5-methylsulfonyl-benzoesäure das N-Diaminomethylen-2-ethyl-4-(4-methoxy-phenoxy)-5-methylsulfonyl-benzamid;
mit 2-Ethyl-4-(2,4-dimethoxy-phenoxy)-5-methylsulfonyl-benzoesäure das N-Diaminomethylen-2-ethyl-4-(2,4-dimethoxy-phenoxy)-5-methylsulfonyl-benzamid;
mit 2-Ethyl-4-(2-methoxy-phenoxy)-5-methylsulfonyl-benzoesäure das N-Diaminomethylen-2-ethyl-4-(2-methoxy-phenoxy)-5-methylsulfonyl-benzamid;
mit 2-Ethyl-4-(3-trifluormethyl-phenoxy)-5-methylsulfonyl-benzoesäure das N-Diaminomethylen-2-ethyl-4-(3-trifluormethyl-phenoxy)-5-methylsulfonyl-benzamid;
mit 2-Ethyl-4-(4-trifluormethyl-phenoxy)-5-methylsulfonyl-benzoesäure das N-Diaminomethylen-2-ethyl-4-(4-trifluormethyl-methylsulfonyl-benzamid;
mit 2-Ethyl-4-(2-trifluormethyl-phenoxy)-5-methylsulfonyl-benzoesäure das N-Diaminomethylen-2-ethyl-4-(2-trifluormethyl-phenoxy)-5-methylsulfonyl-benzamid;
mit 2-Ethyl-4-(3-methyl-phenoxy)-5-methylsulfonyl-benzoesäure das N-Diaminomethylen-2-ethyl-4-(3-methyl-phenoxy )-5-methylsulfonyl-benzamid;
mit 2-Ethyl-4-(4-methyl-phenoxy)-5-methylsulfonyl-benzoesäure das N-Diaminomethylen-2-ethyl-4-(4-methyl-phenoxy)-5-methylsulfonyl-benzamid;
mit 2-Ethyl-4-(2,4-dimethyl-phenoxy)-5-methylsulfonyl-benzoesäure das N-Diaminomethylen-2-ethyl-4-(2,4-dimethyl-phenoxy)-5-methylsulfonyl-benzamid;
mit 2-Ethyl-4-(2-methyl-phenoxy)-5-methylsulfonyl-benzoesäure das N-Diaminomethylen-2-ethyl-4-(2-methyl-phenoxy)-5-methylsulfonyl-benzamid.

### Beispiel 7

600 mg N-Diaminomethylen-2-methyl-4-methoxy-5-methylsulfonyl-benzamid [erhältlich nach Bsp. 2] werden in 80 ml Pyridin zusammen mit 1 g Pyridinhydrochlorid 3 Stunden gekocht. Man kühlt ab, dampft ein, arbeitet wie üblich auf und erhält N-Diaminomethylen-2-methyl-4-hydroxy-5-methyl-sulfonyl-benzamid, F. > 260°.

### Beispiel 8

1,1 g N-Diaminomethylen-2-ethyl-4-benzyloxy-5-methylsulfonyl-benzamid [erhältlich nach Bsp. 6] werden in 30 ml Toluol gelöst und bei Raumtemperatur eine Stunde mit Wasserstoffgas (p = 1 atm) unter katalytischer Wirkung von 150 mg Pd-Kohle (Pd-Gehalt 1%) behandelt. Anschließend filtriert man die Reaktionsmischung und erhält nach üblicher Aufarbeitung N-Diaminomethylen-2-ethyl-4-hydroxy-5-methylsulfonyl-benzamid.

### Beispiel 9

Analog Beispiel 3 erhält man durch Umsetzung mit 1 n Methansulfonat-Lösung die folgenden Methansulfonate:
N-Diaminomethylen-2-methyl-4-ethoxy-5-methylsulfonyl-benzamid, Methansulfonat;
N-Diaminomethylen-2-methyl-4-isopropoxy-5-methylsuffonyl-benzamid, Methansulfonat;
N-Diaminomethylen-2-methyl-4-cyclopentoxy-5-methylsulfonyl-benzamid, Methansulfonat;
N-Diaminomethylen-2-methyl-4-cyclohexyloxy-5-methylsulfonyl-benzamid, Methansulfonat;
N-Diaminomethylen-2-methyl-4-benzyloxy-5-methylsulfonyl-benzamid, Methansulfonat;
N-Diaminomethylen-2-methyl-4-phenoxy-5-methylsulfonyl-benzamid, Methansulfonat, F. 260-262°;
N-Diaminomethylen-2-methyl-4-tert.-butoxy-5-methylsulfonyl-benzamid, Methansulfonat, F. 164-167°;
N-Diaminomethylen-2-methyl-4-(3-chlorphenoxy)-5-methylsulfonyl-benzamid, Methansulfonat, F. > 144°;
N-Diaminomethylen-2-methyl-4-hydroxy-5-methylsulfonyl-benzamid, Methansulfonat, amorph;
N-Diaminomethylen-2-methyl-4-(4-chlorphenoxy)-5-methylsulfonyl-benzamid, Methansulfonat, F. 260°;
N-Diaminomethylen-2-methyl-4-(2-chlorphenoxy)-5-methylsulfonyl-benzamid, Methansulfonat, F. 217-219°;

### Beispiel 10

Analog Beispiel 1 erhält man durch Umsetzung von 2-Methyl-4-(3-propen-1-yl)-5-methylsulfonyl-benzoesäuremethylester mit Guanidin das N-Diaminomethylen-2-methyl-4-(3-propen-1-yl)-5-methylsulfonyl-benzamid, F. 207-209°.

Das entsprechende Methansulfonat wird in Analogie zu Beispiel 9 erhalten und schmilzt bei 187-189°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatrium-hydrogenphosphat werden in 3 1 zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Benzoylguanidin-Derivate der Formel I 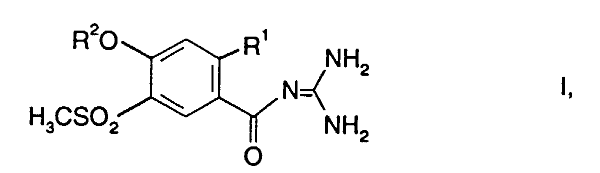 worin
R¹ Methyl oder Ethyl und
R² H, einen geradkettigen oder verzweigten C₁-C₆-Alkyl- oder Alkenyl-Rest, C₃-C₇-Cycloalkyl, Benzyl oder unsubstituiertes oder ein-, zwei- oder dreifach durch Methyl, Methoxy, Amino, F, Cl, Br oder CF₃ substituiertes Phenyl
bedeuten,
sowie deren physiologisch unbedenkliche Salze.

2. (a) N-Diaminomethylen-2-methyl-4-phenoxy-5-methylsulfonyl-benzamid;
(b) N-Diaminomethylen-2-ethyl-4-methoxy-5-methylsulfonyl-benzamid;
(c) N-Diaminomethylen-2-methyl-4-ethoxy-5-methylsulfonyl-benzamid;
(d) N-Diaminomethylen-2-methyl-4-(2-chlorphenoxy)-5-methyl-sulfonylbenzamid;
(e) N-Diaminomethylen-2-methyl-4-(4-chlorphenoxy)-5-methyl-sulfonylbenzamid;
gemäß Anspruch 1, sowie deren physiologisch unbedenkliche Salze.

3. Verfahren zur Herstellung von Benzoylguanidin-Derivaten der Formel I gemäß Anspruch 1 sowie von deren Salzen, dadurch gekennzeichnet, daß man eine Verbindung der Formel II 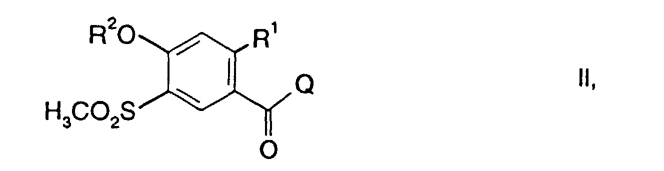 worin R¹ und R² die zuvor angegebenen Bedeutungen haben
und
Q Cl, Br, OA, O-CO-A, O-CO-Ph, OH oder eine andere reaktionsfähige veresterte OH-Gruppe bzw. leicht nucleophil substituierbare Abgangsgruppe und
A Alkyl mit 1-6 C-Atomen bedeutet,
mit Guanidin umsetzt,
oder daß man ein Benzoylguanidin der Formel III 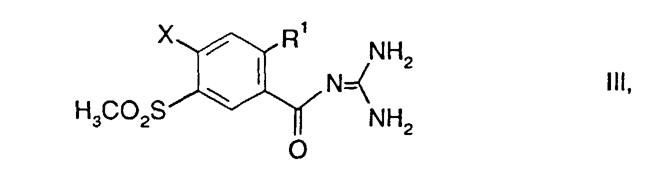 worin R¹ die zuvor angegebenen Bedeutungen besitzt,
und
X F, Cl, Br, I oder eine andere geeignete Abgangsgruppe bedeutet,
mit einem Alkohol bzw. Phenol der Formel IV
R²-OH IV
worin
R² die angegebene Bedeutung besitzt,
gegebenenfalls nach vorheriger Überführung in ein Alkoholat,
umsetzt,
oder daß man einen Alkohol (Phenol) der Formel I, worin R² = H und R¹ die angegebene Bedeutung besitzt, mit einer Verbindung der Formel R²-X, worin R² und X die angegebenen Bedeutungen besitzen, umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man eine erhaltene Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

7. Verwendung von Verbindungen der Formel I nach Anspruch 1 zur Herstellung von Arzneimitteln zur Behandlung von Arrhythmien, Angina pectoris, Infarkten sowie zur präventiven Behandlung der genannten Indikationen.

## Claims

1. Benzoylguanidine derivatives of the formula I 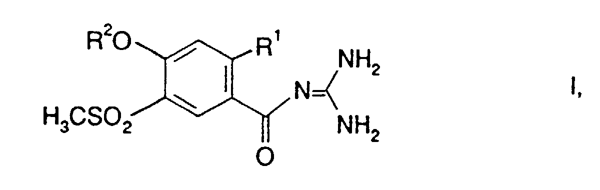 wherein
R¹ is methyl or ethyl and
R² is H, a straight-chain or branched C₁-C₆-alkyl or alkenyl radical, C₃-C₇-cycloalkyl, benzyl or phenyl which is unsubstituted or mono-, di- or trisubstituted by methyl, methoxy, amino, F, Cl, Br or CF₃,
and their physiologically acceptable salts.

2. (a) N-Diaminomethylene-2-methyl-4-phenoxy-5-methylsulfonylbenzamide;
(b) N-diaminomethylene-2-ethyl-4-methoxy-5-methylsulfonylbenzamide;
(c) N-diaminomethylene-2-methyl-4-ethoxy-5-methylsulfonylbenzamide;
(d) N-diaminomethylene-2-methyl-4-(2-chlorophenoxy)-5-methylsulfonylbenzamide;
(e) N-diaminomethylene-2-methyl-4-(4-chlorophenoxy)-5-methylsulfonylbenzamide;
according to Claim 1, and their physiologically acceptable salts.

3. Process for the preparation of benzoylguanidine derivatives of the formula I according to Claim 1, and also their salts, characterized in that a compound of the formula II 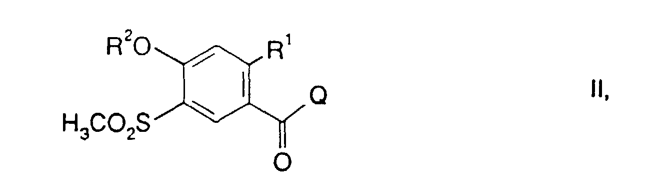 wherein R¹ and R² have the meanings indicated above
and
Q is Cl, Br, OA, O-CO-A, O-CO-Ph, OH or another reactive esterified OH group or leaving group which can be easily nucleophilically substituted and
A is alkyl having 1-6 C atoms,
is reacted with guanidine,
or in that a benzoylguanidine of the formula III 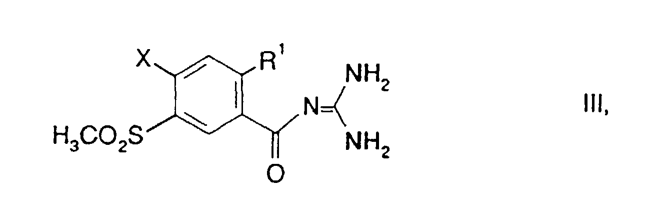 wherein R¹ has the meanings indicated above,
and
X is F, Cl, Br, I or another suitable leaving group,
is reacted with an alcohol or phenol of the formula IV
R²-OH IV
wherein
R² has the meaning indicated,
if appropriate after prior conversion to an alkoxide,
or in that an alcohol (phenol) of the formula I wherein R² = H and R¹ has the meaning indicated is reacted with a compound of the formula R²-X wherein R² and X have the meanings indicated,
or in that a compound which otherwise corresponds to the formula I, but which instead of one or more hydrogen atoms contains one or more reducible groups and/or one or more additional C-C and/or C-N bonds, is treated with a reducing agent,
or in that a compound which otherwise corresponds to the formula I, but which instead of one or more hydrogen atoms contains one or more solvolysable groups, is treated with a solvolysing agent
and/or in that a base of the formula I which is obtained is converted into one of its salts by treating with an acid.

4. Process for the production of pharmaceutical preparations, characterized in that a compound of the formula I according to Claim 1 and/or one of its physiologically acceptable salts is brought into a suitable dose form together with at least one solid, liquid or semi-liquid excipient or auxiliary.

5. Pharmaceutical preparation, characterized in that it contains at least one compound of the general formula I according to Claim 1 and/or one of its physiologically acceptable salts.

6. Use of compounds of the formula I according to Claim 1 or of their physiologically acceptable salts for the production of a medicament.

7. Use of compounds of the formula I according to Claim 1 for the production of medicaments for the treatment of arrhythmias, angina pectoris, infarcts and also for the preventive treatment of the indications mentioned.

## Revendications

1. Dérivés de benzoylguanidine de formule I 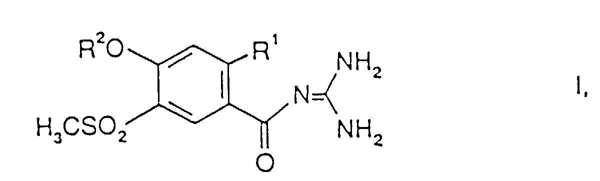 dans laquelle
R¹ représente un méthyle ou un éthyle et
R² représente H, un radical alkyle ou alcényle en C₁ à C₆ à chaîne droite ou ramifiée, un cycloalkyle en C₃ à C₇, un benzyle ou un phényle non substitué ou mono-, di- ou trisubstitué par un méthyle, un méthoxy, un amino, F, Cl, Br, ou CF₃,
ainsi que leurs sels physiologiquement acceptables.

2. (a) N-diaminométhylène-2-méthyl-4-phénoxy-5-méthyl-sulfonyl-benzamide ;
(b) N-diaminométhylène-2-éthyl-4-m,éthoxy-5-méthyl-sulfonylbenzamide ;
(c) N-diaminométhylène-2-méthyl-4-éthoxy-5-méthyl-sulfonylbenzamide ;
(d) N-diaminométhylène-2-méthyl-4- (2-chlorophénoxy) -5-méthyl-sulfonylbenzamide;
(e) N-diaminométhylène-2-méthyl-4-(4-chlorophénoxy)-5-méthyl-sulfonylbenzamide;
selon la revendication 1,
ainsi que leurs sels physiologiquement acceptables.

3. Procédé de préparation de dérivés de benzoylguanidine de formule I selon la revendication 1 ainsi que de leurs sels, caractérisé en ce qu'on fait réagir un composé de formule II 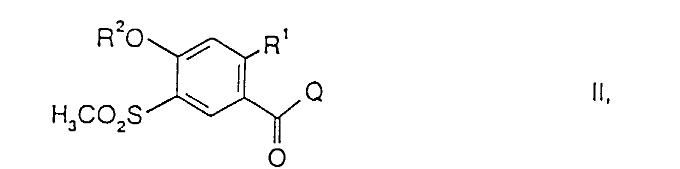 dans laquelle R¹ et R² ont les significations indiquées ci-dessus,
et
Q représente Cl, Br, OA, O-CO-A, O-CO-Ph, OH ou un autre groupe OH estérifié réactif ou selon les cas un groupe sortant substituable légèrement nucléophile et
A représente un alkyle ayant de 1 à 6 atomes de carbone,
avec de la guanidine,
ou en ce qu'on fait réagir une benzoylguanidine de formule III 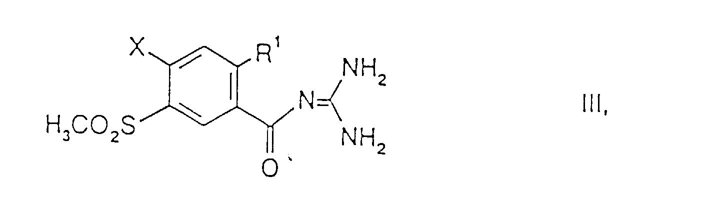 dans laquelle R¹ a la signification indiquée ci-dessus,
et
X représente F, Cl, Br, I ou un autre groupe sortant, avec un alcool ou selon les cas un phénol de formule IV
R²-OH IV
dans laquelle
R² a la signification indiquée ci-dessus,
le cas échéant après transformation préalable en un alcoolate,
ou en ce qu'on fait réagir un alcool (phénol) de formule I, dans laquelle R² = H et R¹ a la signification indiquée, avec un composé de formule R²-X, dans laquelle R² et X ont les significations indiquées,
ou en ce qu'on traite avec un agent réducteur un composé correspondant sinon à la formule I, qui contient cependant au lieu d'un ou plusieurs atomes d'hydrogène un ou plusieurs groupes réductibles et/ou une ou plusieurs liaisons C-C et/ou C-N additionnelles, ou en ce qu'on traite avec un agent de solvolyse un composé correspondant sinon à la formule I, qui contient cependant à la place d'un ou plusieurs atomes d'hydrogènes, un ou plusieurs groupes solvolysables,
et/ou en ce qu'on transforme une base de formule I obtenue par traitement avec un acide en un de ses sels.

4. Procédé de préparation de préparations pharmaceutiques caractérisé en ce qu'on met sous une forme d'administration appropriée un composé de formule I selon la revendication 1 et/ou un de ses sels physiologiquement acceptables avec au moins un support ou additif solide, liquide ou semi-liquide.

5. Préparation pharmaceutique caractérisée en ce qu'elle contient au moins un composé de formule générale I selon la revendication 1 et/ou un de ses sels physiologiquement acceptables.

6. Utilisation de composés de formule I selon la revendication 1 ou de leurs sels physiologiquement acceptables pour la préparation d'un médicament.

7. Utilisation de composés de formule I selon la revendication 1 pour la préparation de médicaments visant à traiter les arythmies, les angines de poitrine, les infarctus, ainsi que pour le traitement préventif des indications mentionnées.
